# EUROPEAN PATENT APPLICATION

(11) **EP 4 775 588 A1**
(43) Date of publication of application: **15.07.2026**
(21) Application number: 25840334.4
(22) Date of filing: 14.07.2025
(51) Int. Cl.: C07K 1/14, A61K 38/01, A61P 25/00

(54) **PROTEIN POLYMER AND PRODUCTION PROCESS THEREFOR**

(30) Priority: 15.07.2024 CN 202410944325
(71) Applicant: Darwin Biotechnology (Hubei) Co., Ltd., Wuhan, Hubei 430075 (CN)
(72) Inventor: WANG, Yu, Wuhan, Hubei 430075 (CN); LI, Fuluan, Wuhan, Hubei 430075 (CN); LIU, Mi, Wuhan, Hubei 430075 (CN)
(74) Representative: Dolphin, Kirsty Mairi
(86) International application number: PCT/CN2025/108488
(87) International publication number: WO 2026/017015

(57) **Abstract**

The present disclosure belongs to the field of biotechnology and discloses a protein complex and production process thereof. The protein complex is obtained by stimulation of MSCs followed by lysis, and then isolation and purification. The protein complex has a strong ability to repair oxidative damage, which can reduce the size of cerebral infarction, improve neurological function, inhibit the level of neuroinflammation, and increase the number of surviving neurons.

## Description

The disclosure claims the priority of Chinese patent application No. 202410944325.9 filed with the China National Intellectual Property Administration on July 15, 2024, and entitled "Protein Complex and Production Process Thereof", which is incorporated herein by reference in its entirety.

### TECHNICAL FIELD

The present disclosure belongs to the field of biotechnology, and specifically relates to protein complex and production process thereof.

### BACKGROUND

Mesenchymal stem cells (MSCs) have the potential of self-replication and multi-directional differentiation, and are widely found in bone marrow, fat, synovium, dental pulp, amniotic fluid, placenta, umbilical cord, embryo, umbilical cord blood, amniotic membrane, peripheral blood, muscle, urine and other tissues, and have the characteristics of wide source, no need for mating, low rate of infection, high differentiation potential, strong proliferation ability, convenient collection, etc. They can produce stem cell growth factor (SCF), nerve growth factor (NGF), interleukin-6 (IL-6), interleukin-7 (IL-7), tumor necrosis factor (TNF), interferon (IFN) and other active factors, which are involved in the regulation of cell growth, apoptosis, cell differentiation, anti-virus, immune maturation and other processes, and can be used for immune regulation, tissue repair and treatment of acute lung injury, severe pneumonia, acute respiratory distress syndrome and other diseases.

MSCs cultured under different stimulatory conditions can produce different stress proteins, and these stress protein complexes have complex physiological activities. How to utilize MSCs to produce a protein complex with specific biological activities is a highly challenging task.

### SUMMARY

It is an object of the present disclosure to provide a protein complex and a production process thereof for overcoming at least one of the deficiencies of the prior art.

The technical solutions adopted in the present disclosure are as follows.

A first aspect of the present disclosure provides: a protein complex, wherein its production process comprises:
S1) culturing mesenchymal stem cells and creating a stressful environment using ultraviolet (UV) irradiation; and
S2) lysing the mesenchymal stem cells, followed by isolation and purification to obtain the protein complex.

In some embodiments of the protein complex, the protein complex comprises at least the following proteins:
sp|P02768|ALBU_HUMAN Serum albumin OS=Homo sapiens; and
sp|P02787|TRFE_HUMAN Serotransferrin OS=Homo sapiens.

Preferably, the mass of the above two proteins accounts for over 40% of the total protein complex mass.

Preferably, the content of Serum albumin protein accounts for at least 38% of the total protein complex content, and the content of Serotransferrin protein accounts for at least 2% of the total protein complex content.

Preferably, the protein complex further comprises at least one of the following proteins:
sp|P51884|LUM_HUMAN Lumican OS=Homo sapiens;
sp|P62736|ACTA_HUMAN Actin, aortic smooth muscle OS=Homo sapiens;
sp|P01009|A1AT_HUMAN Alpha-1-antitrypsin OS=Homo sapiens;
sp|P07951|TPM2_HUMAN Tropomyosin beta chain OS=Homo sapiens;
sp|P08670|VIME_HUMAN Vimentin OS=Homo sapiens;
sp|P02751|FINC_HUMAN Fibronectin OS=Homo sapiens;
sp|P09493|TPM1 _HUMAN Tropomyosin alpha-1 chain OS=Homo sapiens;
sp|P21333|FLNA_HUMAN Filamin-A OS=Homo sapiens;
sp|P0DOX5|IGG1_HUMAN Immunoglobulin gamma-1 heavy chain OS=Homo sapiens;
sp|P24821|TENA_HUMAN Tenascin OS=Homo sapiens;
sp|P01023|A2MG_HUMAN Alpha-2-macroglobulin OS=Homo sapiens;
sp|P60709|ACTB_HUMAN Actin, cytoplasmic 1 OS=Homo sapiens;
sp|P69891|HBG1_HUMAN Hemoglobin subunit gamma-1 OS=Homo sapiens; and
sp|P01024|C3 HUMAN Complement C3 OS=Homo sapiens.

In some embodiments of the protein complex, the UV irradiation is conducted to stimulate the mesenchymal stem cells for 1 h to 30 h, preferably, for 10 h to 30 h, and more preferably for 6 h to 18 h.

Preferably, the UV irradiation stimulation is conducted at an intensity of 10µW/cm² to 100µW/cm², and with a UV wavelength of preferably 290 nm to 340 nm.

In a preferred embodiment, the UV wavelength is 290 nm to 325 nm.

In a specific and preferred embodiment, the UV wavelength is 300 nm to 320 nm.

In a specific and preferred embodiment, the UV wavelength is 300 nm to 316 nm.

Preferably, a medium used during UV irradiation stimulation is serum-free MSCs medium.

In some embodiments of the protein complex, the mesenchymal stem cells are selected from the group consisting of umbilical cord-derived human mesenchymal stem cells, bone marrow-derived mesenchymal stem cells, and human placental-derived mesenchymal stem cells.

In some embodiments of the protein complex, the mesenchymal stem cells are selected from the group consisting of human umbilical cord mesenchymal stem cells, and human amniotic membrane mesenchymal stem cells.

In some embodiments of the protein complex, the protein complex is derived from intracellular components.

In some embodiments of the protein complex, the protein complex is derived from the culture supernatant after stem cell culture.

In some embodiments of the protein complex, the protein complex is derived from the culture supernatant and intracellular components after stem cell culture.

In some embodiments of the protein complex, the lysis is performed with pure water.

The above features can be combined in any way without conflict.

A second aspect of the present disclosure provides: a production process of a protein complex comprising expanding MSCs, subjecting MSCs to a stress treatment through UV irradiation in culture, collecting the stress-treated MSCs for lysis treatment, and isolating and purifying proteins to obtain the protein complex.

In some embodiments of the production process, the UV irradiation is conducted to stimulate the mesenchymal stem cells for 1 h to 30 h, preferably, for 10 h to 30 h, and more preferably for 6 h to 18 h;
preferably, the UV irradiation stimulation is conducted at an intensity of 10µW/cm² to 100µW/cm², and with a UV wavelength of preferably 290 nm to 340 nm;
preferably, a medium used during UV irradiation stimulation is serum-free MSCs medium.

The above features can be combined in any way without conflict.

A third aspect of the present disclosure provides: use of the protein complex of the first aspect of the present disclosure, wherein the use comprises the use in the preparation of a medicament for the treatment of neurodegenerative diseases, and stroke. Furthermore, the neurodegenerative diseases include, but are not limited to, Alzheimer's Disease (AD), Parkinson's Disease (PD), amyotrophic lateral sclerosis (ALS), and different types of Spinocerebellar Ataxia (SCA).

The beneficial effects of the present disclosure are as follows:
the protein complex in some embodiments of the present disclosure, which has favorable repair effects for cellular injury, are expected to be used for the treatment of neurodegenerative diseases, and strokes, and in particular, the neurodegenerative diseases include, but are not limited to, Alzheimer's Disease (AD), Parkinson's Disease (PD), Amyotrophic Lateral Sclerosis (ALS), and different types of Spinocerebellar Ataxia (SCA).

The production process in some embodiments of the present disclosure can effectively overcome the differences between different batches of MSCs and obtain more stable MSCs with less batch-to-batch variations, greatly ensuring the quality and yield of the protein complex.

The production process in some embodiments of the present disclosure can better ensure the activity of umbilical cord-derived MSCs and BMSCs and are conducive to increasing the original acquisition quantity of MSCs.

The production process in some embodiments of the present disclosure has a high rate of cryopreservation and resuscitation viability of MSCs.

The production process in some embodiments of the present disclosure allows for good isolation and purification of the protein complex.

### BRIEF DESCRIPTION OF THE DRAWINGS

Figure 1 is a photograph of the growth status of MSCs after incubation in TangYi 3D medium in culture test 1-1.
Figure 2 is a photograph of the cell state after 8h of UV irradiation in culture test 1-1.
Figure 3 shows the SDS-PAGE results of proteins harvested from culture test 1-1.
Figure 4 is a photograph of the cell state of MSCs before UV irradiation in culture test 1-2.
Figure 5 is a photograph of the cell state of MSCs after 6h of low-intensity UV irradiation in culture test 1-2.
Figure 6 shows the SDS-PAGE results of proteins harvested from culture test 1-2.
Figure 7 shows the SDS-PAGE results of intracellular proteins and proteins from the culture supernatant harvested from culture test 1-3.
Figure 8 is a statistical histogram of the repair capacity of the protein complexes obtained from different treatment groups in Experiment II for injured nerve cells.
Figure 9 is a histogram of the percentage of motor neurons with stress granules (SGs) formed by SA injury, treated with the samples obtained from the different treatment groups in Experiment III.
Figure 10 shows the effect of the protein complexes obtained from different treatment groups on the secretion of the inflammatory factor IL-6 by RAW cells in Experiment IV.
Figure 11 is a statistical histogram of the repair capacity of the protein complexes obtained from different treatment groups in Experiment V for injured nerve cells.
Figures 12 and 13 show the effect of different treatments on the size of cerebral infarction in rats.
Figure 14 shows the effect of different treatments on the neurological function of rats.
Figure 15 shows the effects of intrathecal combined intravenous administration of protein complexes on neurons.

### DETAILED DESCRIPTION OF THE EMBODIMENTS

The technical solutions of the present disclosure are further described below in connection with experimental examples.

### Experiment I. Effect of different treatments on protein expression

### 1-1 Culture test I

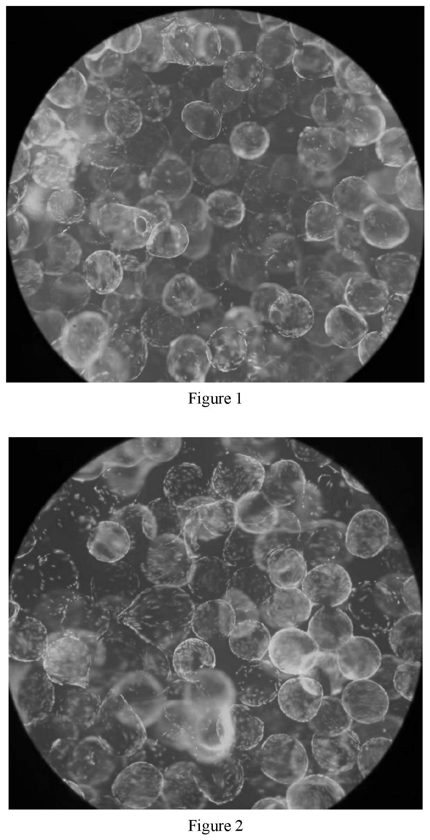

A total of 2 L of human umbilical cord mesenchymal stem cells (HUC-MSC) with a total number of cells of about 5×10⁸ were cultured with HK-G050 (PRF) 3D medium from TangYi Huike Biologicals, and aliquoted into 4 T225 flasks. Cell staining was observed and the microcarriers were essentially covered with the cell. The results are shown in Figure 1.

Cells were irradiated by LED UV light with irradiation condition of 60 µW/cm². Samples were taken at 8 h, 12h, 16h, 18h, 24h, and 30h. Cell morphology was observed, and intracellular proteins were collected to measure the protein concentration.

Taking cells under UV irradiation for 8 h as an example, the cell state after irradiation is shown in Figure 2. Figures 1 and 2 show that the microcarriers were essentially covered with cells at 0 h. Irradiation by UV for a period of time affected the cell morphology and imposed survival stress on the cells, which in turn can produce stress proteins in this stressful environment.

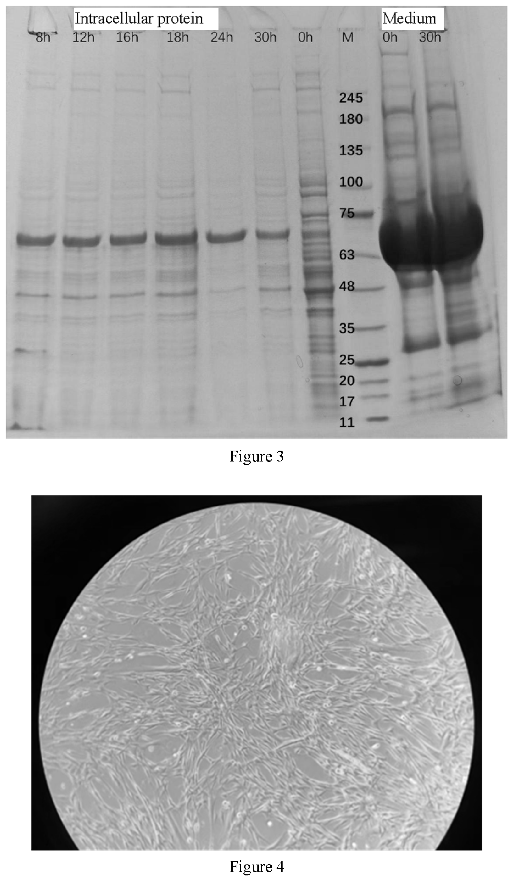

At the end of irradiation, the cells were harvested at different time points to obtain protein complexes. The specific operation was as follows: for the seven groups of 0h, 8h, 12h, 16h, 18h, 24h, and 30h, 8 mL of culture was taken from each, and the following operations were carried out: the culture was passed through a 300-mesh filter bag to intercept the microcarriers. The supernatant was centrifuged at 1200 rpm for 6 min, and stored at 4°C. The cell pellets were washed with 150 mL of saline 3 times, lysed in 14 mL of pure water, and passed through 0.22 µm filter membrane. The harvested proteins were lyophilized at -80°C (if used in a short term, they can be stored at 4°C). The harvested proteins were taken and analyzed by gel electrophoresis. Their SDS-PAGE results are shown in Figure 3.

### 1-2 Culture test II

One small crystal P8 generation HUC-MSC was resuscitated to 9 T25 culture flasks, 2.5 mL of CytoNiche MSC serum-free medium was added to each flask, and the cells were inoculated at a density of 1×10⁵ cells/mL in each culture flask.

Four flasks of cells each were taken and irradiated respectively with two different intensities of UV B-based LED UV at around 300 nm to 316 nm for 6h, 12h, 18h, and 24h, and the irradiation conditions were as follows. The supernatant was carefully removed from the cells and the cells were washed with 1 mL of saline twice, and 660 µL of pure water was added to lyse cells for 10 min by repeatedly pipetting, and then the lysed cells were passed through 0.22 µm filter membrane, and stored at 4°C.

The UV irradiation conditions are as follows:

| Intensity of the UV irradiation | **High-intensity** | **Low-intensity** |
|---|---|---|
| Intermediate medium | T25 flask | T25 flask |
| UVA (µW/cm²) | 17.26 | 11.71 |
| UVB (µW/cm²) | 47.89 | 26.81 |
| UVC (µW/cm²) | 2.75 | 0.99 |
| Peak wavelength (nm) | 308.4 | 308.4 |

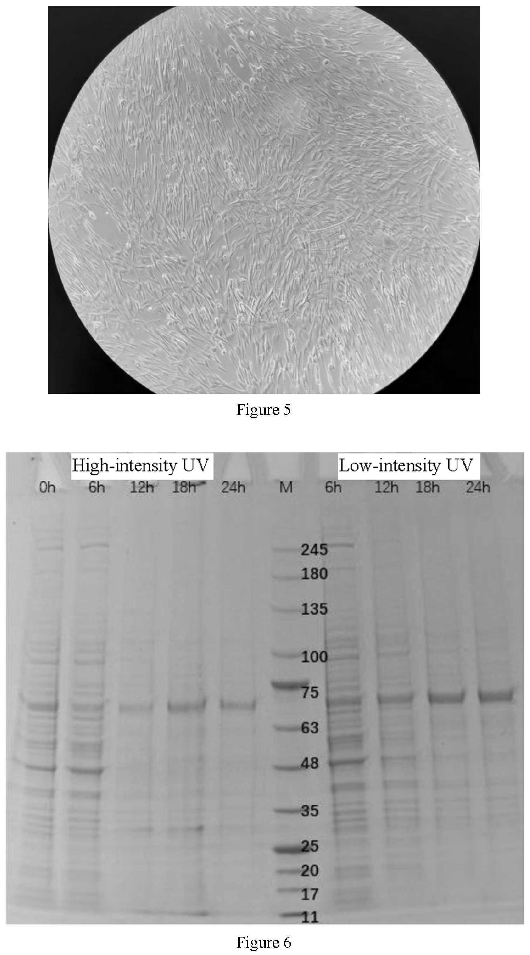

The cell state before UV irradiation is shown in Figure 4, and the cell state after irradiation with low-intensity for 6h is shown in Figure 5, and it can be seen from Figures 4 and 5 that UV irradiation affects the cell morphology, and imposes a survival stress on the cells, and then the cells may produce stress proteins under this stressful environment. Methods for determining protein concentration are conventional methods known to those skilled in the art and include, for example, the Bradford method, the BCA method, the Lowry method, UV spectrophotometry, and the Kjeldahl method. In the present disclosure, the BCA method was adopted to determine the protein concentration at each time point. The concentration and volume of harvested proteins are shown in Table 1.

**Table 1**

| Irradiation time (h) | 0 | 6 | 12 | 18 | 24 |
|---|---|---|---|---|---|
| High-intensity, Protein concentration (mg/mL) | 0.675 | 0.670 | 0.155 | 0.165 | 0.082 |
| Low-intensity, Protein concentration (mg/mL) | 0.681 | 0.718 | 0.343 | 0.215 | 0.165 |
| Volume (µL) | 500 | 500 | 500 | 500 | 500 |

The SDS-PAGE results of the harvested proteins are shown in Figure 6. As shown in Figure 6, both high-intensity and low-intensity UV irradiation conditions can promote the expression of proteins of interest. Under the high-intensity UV irradiation condition, the proteins of interest became purer with the increase of irradiation time. The purity of the proteins of interest was higher and the concentration of the proteins of interest was also higher at about 18h of irradiation. Low-intensity UV irradiation can effectively promote the expression of proteins of interest, but requires a longer irradiation time than that needed for high-intensity UV irradiation.

### 1-3 Culture test III

One small crystal P8 generation HUC-MSC was resuscitated to T25 culture flasks, 2.5 mL of CytoNiche MSC serum-free medium was added to each flask, and the cells were inoculated at a density of 1×10⁵cells/mL in each culture flask.

Cells were taken and irradiated under UVB UV conditions at wavelengths of 300 nm to 316 nm for 6h, 12h, 18h, 24h and 30h.

The UV irradiation conditions are as follows:

| UV irradiation characteristics | |
|---|---|
| UVB (µW/cm²) | 40.05 |
| Peak wavelength (nm) | 306.0 |

At the end of irradiation, cells were harvested at different time points to obtain protein complexes. The specific operation was as follows: for irradiation of 6h, 12h, 18h, 24h and 30h, the culture supernatant was taken at each time point, and the following operations were carried out: the supernatant was passed through 0.22 µm filter membrane and stored at 4°C.

Then the remaining cells were washed with 2 mL of saline twice, and then 1 mL of pure water was added to repeatedly pipette the cells down from the bottom of the flask, and the cells were lysed by repeatedly pipetting for about 6 min, and then passed through 0.22 µm filter membrane, and stored at 4°C for use.

Harvested proteins were taken for gel electrophoresis analysis. The protein concentrations obtained under each culture condition are shown in Table 2 below:

**Table 2**

| | Intracellular proteins | | | | | | Supernatant proteins | | | | | |
|---|---|---|---|---|---|---|---|---|---|---|---|---|
| Irradiation time (h) | 0 | 6 | 12 | 18 | 24 | 30 | 0 | 6 | 12 | 18 | 24 | 30 |
| Protein concentration (mg/mL) | 0.698 | 0.681 | 0.173 | 0.201 | 0.099 | 0.086 | 3.521 | 3.612 | 3.645 | 3.876 | 3.885 | 3.886 |
| Volume | 500µL | 500µL | 500µL | 500µL | 500µL | 500µL | 2mL | 2mL | 2mL | 2mL | 2mL | 2mL |

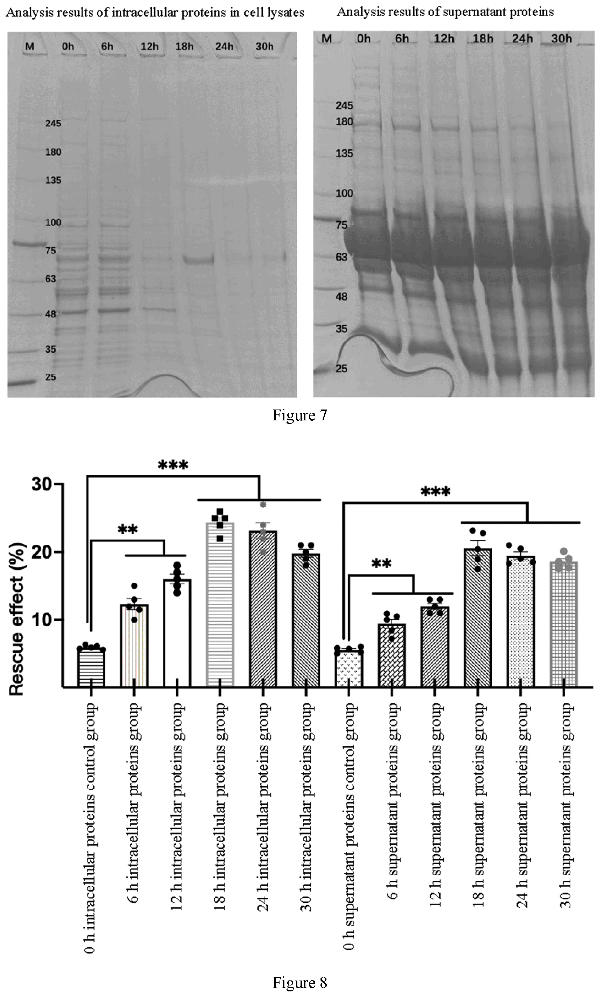

The above harvested intracellular proteins and stem cell culture supernatant proteins were analyzed using SDS-PAGE, respectively, and the results are shown in Figure 7. As shown in Figure 7, the amount of proteins of interest contained in intracellular proteins gradually increased with the extension of irradiation time, and at an irradiation time of about 18h, there were very few impurity proteins and most of the bands were proteins of interest. In the supernatant, it also contains the bands of proteins of interest.

Mass spectrometry analysis was performed on the protein complex described above, and known proteins were matched based on the mass spectrometry data confirming the determination that the protein complex contained the following two proteins:
sp|P02768|ALBU_HUMAN Serum albumin OS=Homo sapiens; and
sp|P02787|TRFE_HUMAN Serotransferrin OS=Homo sapiens;
wherein the content of Serum albumin protein accounts for at least 38% of the total protein complex content, and the content of Serotransferrin protein accounts for at least 2% of the total protein complex content.

Further, in addition to the above two proteins, the protein complex obtained in the present application comprises at least one of the following proteins:
sp|P51884|LUM_HUMAN Lumican OS=Homo sapiens;
sp|P62736|ACTA_HUMAN Actin, aortic smooth muscle OS=Homo sapiens;
sp|P01009|A1AT_HUMAN Alpha-1-antitrypsin OS=Homo sapiens;
sp|P07951|TPM2_HUMAN Tropomyosin beta chain OS=Homo sapiens;
sp|P08670|VIME_HUMAN Vimentin OS=Homo sapiens;
sp|P02751|FINC_HUMAN Fibronectin OS=Homo sapiens;
sp|P09493|TPM1 _HUMAN Tropomyosin alpha-1 chain OS=Homo sapiens;
sp|P21333|FLNA_HUMAN Filamin-A OS=Homo sapiens;
sp|P0DOX5|IGG1_HUMAN Immunoglobulin gamma-1 heavy chain OS=Homo sapiens;
sp|P24821|TENA_HUMAN Tenascin OS=Homo sapiens;
sp|P01023|A2MG_HUMAN Alpha-2-macroglobulin OS=Homo sapiens;
sp|P60709|ACTB_HUMAN Actin, cytoplasmic 1 OS=Homo sapiens;
sp|P69891|HBG1_HUMAN Hemoglobin subunit gamma-1 OS=Homo sapiens; and
sp|P01024|C3 HUMAN Complement C3 OS=Homo sapiens.

### Experiment II. The efficacy test of the protein complex for repair of nerve cell injury

Cell modeling and detection: after 24 hours of inoculation of SH-SY5Y cell on the plate, three subgroups were set up, that is, the normal group, the model group and the administration group. The model and administration groups were injured with 250 µM H₂O₂ for 30 min, then the supernatant was discarded, and the administration group was administered at a protein concentration of 100 ng/mL, with five replicate wells for each sample, and the model and normal cell groups were changed to normal medium. Wherein administration group 1 is the control group of 0 h intracellular protein in test 1-3; wherein administration group 2 is the 6 h intracellular protein group in test 1-3; wherein administration group 3 is the 12 h intracellular protein group in test 1-3; wherein administration group 4 is the 18 h intracellular protein group in test 1-3; wherein administration group 5 is the 24 h intracellular protein group in test 1-3; wherein administration group 6 is the 30 h intracellular protein group in test 1-3; wherein administration group 7 is the 0 h supernatant protein control group in test 1-3; wherein administration group 8 is the 6 h supernatant protein group in test 1-3; wherein administration group 9 is the 12 h supernatant protein group in test 1-3; wherein administration group 10 is the 18 h supernatant protein group in test 1-3; wherein administration group 11 is the 24 h supernatant protein group in test 1-3; and wherein administration group 12 is the 30 h supernatant protein group in test 1-3. SH-SY5Y nerve cells were further cultured for 72 h. Cell viability was detected by CellTiter-glo luminescence assay (cell viability was detected by chemiluminescence assay at 590 nm using Biyoungtian CellTiter-Lµmi^{™} II Cell Activity Assay Kit).

The experimental results are shown in Figure 8, the intracellular proteins and supernatant proteins of the stem cells without undergoing UV irradiation (0 h) have certain nerve cell protective function, but their protective ability is very weak. In contrast, intracellular proteins and supernatant proteins of the stem cells cultured for different periods of time under UV irradiation conditions have strong protective ability of nerve cell repair. In particular, intracellular proteins and secretory proteins secreted into the supernatant from stem cells cultured under UV irradiation conditions for more than 12 h have the strongest protective ability of nerve cells.

### Experiment III. The efficacy of the protein complex on the formation of stress granules (SG) in motor neurons injured by sodium arsenite (SA)

Intracellular proteins and supernatant proteins from cell lysates of MSCs cultured under normal conditions for 18 h without UV irradiation were prepared using the same experimental method as that in Experiment II. At the same time, intracellular proteins and supernatant proteins from cell lysates of MSCs that were UV irradiated for 18 h under the conditions described in Experiment II were prepared. At the same time, simple stem cell culture medium and albumin control samples were also prepared with UV irradiation and without UV irradiation, respectively. The information on the samples obtained is shown in Table 3:

**Table 3. Experimental sample information**

| **Sample Code** | **Sample Information** |
|---|---|
| 1 | Intracellular proteins of cell lysates from stem cells without UV irradiation (18 h) |
| 2 | Intracellular proteins of cell lysates from stem cells with UV irradiation (18 h) |
| 3 | Culture supernatant proteins of stem cells without UV irradiation (18 h) |
| 4 | Culture supernatant proteins of stem cells with UV irradiation (18 h) |
| 5 | Simple stem cell culture medium without irradiation |
| 6 | Simple stem cell culture medium with UV irradiation (18 h) |
| 7 | Human serum albumin without UV irradiation |
| 8 | Human serum albumin with UV irradiation (18 h) |

The experimental procedure for the efficacy test of each of the above samples was as follows:
Primary motor neurons (MN) were cultured to day 7 (DIV7) and subjected to sodium arsenite (SA)-induced injury according to the following protocol:
(1) Model group (SA group): following injured by 400 µM of SA for 30 min, the medium was replaced with complete medium, and the cells were cultured for 1.5 h;
(2) Administration group: following injured by 400 µM of SA for 30 min, the cells were added into the medium containing Sample 1, or Sample 2, or Sample 3, or Sample 4, or Sample 5, or Sample 6, or Sample 7, or Sample 8, and cultured for 1.5 h;
(3) Normal control group (Ctr group): the cells were treated with normal medium in parallel processing in the whole procedure.

After the cells were fixed, the stress granules were labeled with immunofluorescence staining. Photographs were taken with a fluorescence microscope, the number of G3BP1 fluorescent particles (stress granules) in neurons labeled as NeuN-positive were analyzed with ImageJ, SG particles were counted for MNs in random fields of view in different groups, and Graphpad was used to do a total score-percentage count, and histograms were made to summary the results.

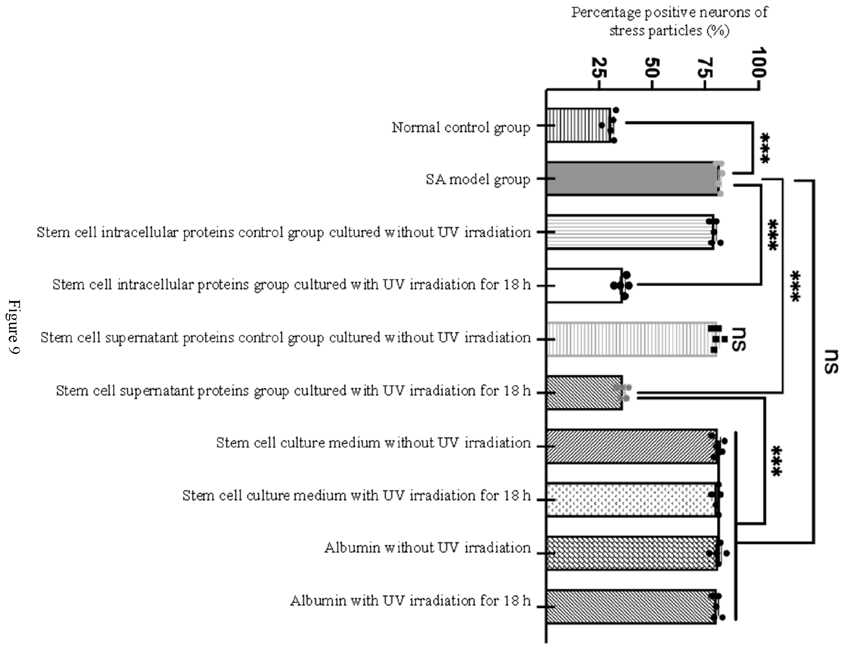

Experimental results: as shown in Figure 9, SA stimulation treatment can increase the content of stress granules in nerve cells compared to normal control. In contrast, intracellular proteins and supernatant proteins of stem cells with UV irradiation can repair increased stress granules within neurons induced by SA. However, the sample from each control group does not have the function of protecting and repairing neurons. The experimental results suggest that UV-irradiated MSCs produce specific proteins that attenuate neuronal damage caused by pathologic aggregation of SGs and have therapeutic potential for nerve cell damage or related neurodegenerative diseases.

### Experiment IV. Protein products obtained from mesenchymal stem cells cultured with UV irradiation have anti-inflammatory effects

Human umbilical cord MSCs or human amniotic MSCs were cultured under UV B irradiation for 18 h according to the experimental conditions and experimental methods described in test 1-3, respectively, and then the culture supernatant was taken, and the supernatant was passed through 0.22 µL filter membrane and stored at 4°C. Then the remaining cells were washed with 2 mL of saline twice, and then 1 mL of pure water was added to repeatedly pipette the cells down from the bottom of the flask, and the cells were lysed by repeatedly pipetting for about 6 min, and then passed through 0.22 µm filter membrane, and stored at 4°C for use.

RAW cells are a common inflammatory cell model. RAW cells were used as inflammation model cells used in this experiment. Firstly, they were plated in a 96-well plate at 20,000 RAW cells/well. Then the inflammatory cell models were established by stimulating RAW cells for 24h using LPS (500 ng/mL). The LPS supernatant was then aspirated and discarded, each of samples were added (corresponding samples of 500 ng/mL protein content), and fresh medium was added to the model group. After 24h, cell supernatant was collected. The supernatant was diluted at 15-fold to 20-fold and then the level of IL-6 was tested according to the instructions of the ELISA kit.

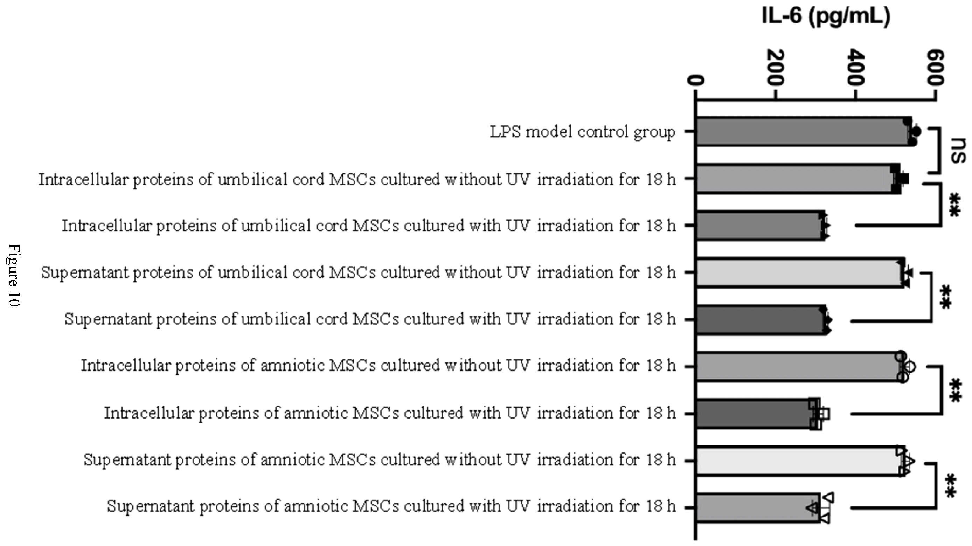

IL-6 is the most common inflammatory factor, which rises in response to inflammation, and the ability to reduce IL-6 levels indicates anti-inflammatory function. The experimental results are shown in Figure 10. The intracellular proteins and the supernatant proteins of the umbilical cord MSCs or amniotic membrane MSCs described in the present disclosure after UV irradiation stress culture for a certain period of time can inhibit the inflammatory response in the model cells and have an anti-inflammatory function.

### Experiment V. Intracellular or supernatant protein products from amniotic membrane mesenchymal stem cells cultured by UV irradiation have nerve cell repair functions

Human amniotic MSCs were collected according to conventional methods. The abbreviated steps were as follows: amniotic membrane tissue was isolated from human placental amniotic membrane, cut into pieces with surgical scissors, and primary amniotic membrane MSCs were isolated and cultured by tissue adherent method. After 5 days of tissue adherent culture, a large number of primary cells migrated out, and trypsin digestion was used for cell passaging, and when the cell confluency reached 80% to 90%, the passaging was carried out according to the density of 3,000 cells/cm², then the human amniotic MSCs were obtained. Cells were cryopreserved at 5×10⁶ cells/tube for use.

Human amniotic MSCs were cultured with UVB irradiation for 18 h or without UV irradiation for 18 h (i.e., the 0 h group in test 1-3) according to the experimental conditions and experimental methods described in test 1-3, and then the culture supernatant was taken respectively, and the supernatant was passed through 0.22 µL filter membrane, and stored at 4°C. Then the remaining cells were washed with 2 mL of saline twice, and then 1 mL of pure water was added to repeatedly pipette the cells down from the bottom of the flask, and the cells were lysed by repeatedly pipetting for about 6 min, and then passed through 0.22 µm filter membrane, and stored at 4°C for use.

Cell modeling and detection: after 24 hours of inoculation of SH-SY5Y cell on the plate, three subgroups were set up, that is, the normal group, the model group and the administration group. The model and administration groups were injured with 250 µM H₂O₂ for 30 min, then the supernatant was discarded, and the administration group was administered at a protein concentration of 100 ng/mL, with five replicate wells for each sample, and the model and normal cell groups were changed to normal medium. Wherein administration group 1 is the intracellular protein control group obtained from MSCs cultured without UV irradiation for 18 h (i.e., group 0 h); wherein administration group 2 is the intracellular protein group obtained from MSCs cultured with UV irradiation for 18 h; wherein administration group 3 is the supernatant protein control group obtained from MSCs cultured without UV irradiation for 18 h (i.e., group 0 h); and wherein administration group 4 is the supernatant protein group obtained from MSCs cultured with UV irradiation for 18 h. SH-SY5Y nerve cells were further cultured for 72 h. Cell viability was detected by CellTiter-glo luminescence assay (cell viability was detected by chemiluminescence assay at 590 nm using Biyoungtian CellTiter-Lµmi^{™} II Cell Activity Assay Kit).

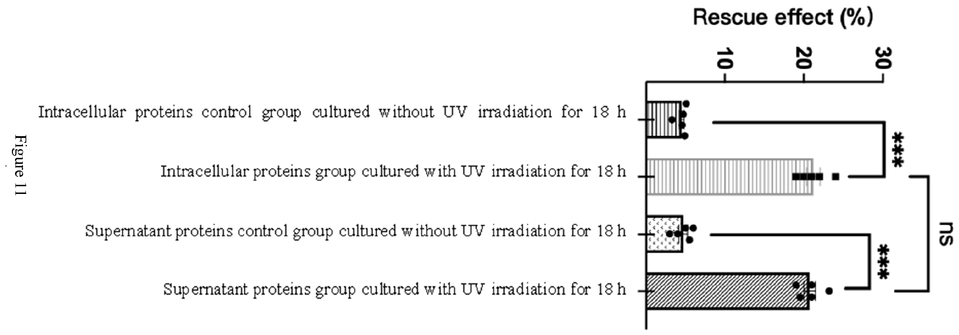

The experimental results are shown in Figure 11, the intracellular proteins or supernatant proteins of the stem cells without undergoing UV irradiation (the group cultured for 18 h without UV irradiation) have certain nerve cell protective function, but their protective ability is very weak. In contrast, intracellular proteins or supernatant proteins of the stem cells cultured under UV irradiation conditions have strong protective ability of nerve cell repair.

### Experiment VI. Use of protein complexes in stroke therapy

The following protein complex obtained from Experiment I of the present disclosure were used to further investigate their biological activity.

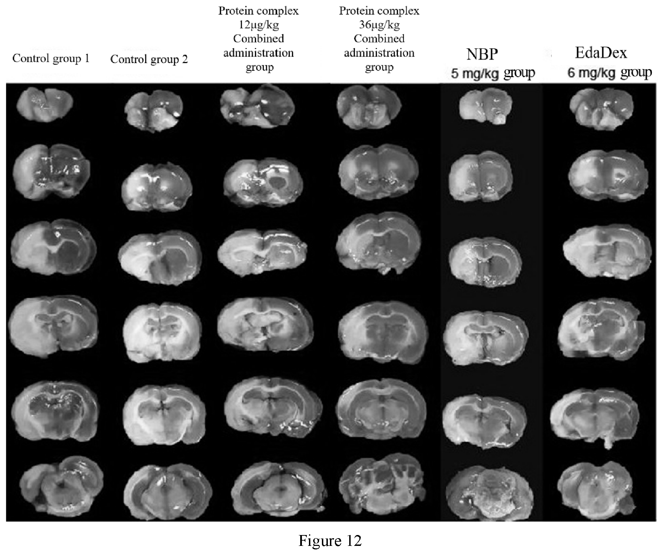

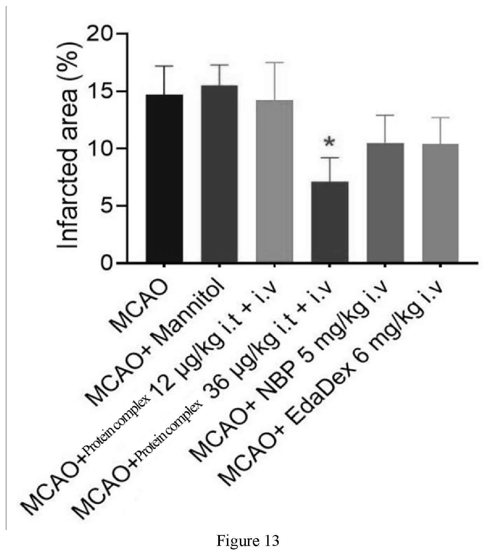

Cerebral infarction was observed by TTC staining after 7 days of administration to MCAO rats, and the intrathecal combined intravenous administration of 36 µg/kg of protein complex (protein complex obtained from Culture test II) significantly reduced the size of cerebral infarction in the rats in the group (p<0.05), whereas the administration of either Butylphthalide (NBP) or Edaravone and Dexborneol (EdaDex) failed to significantly reduce the size of cerebral infarction (Figures 12 and 13).

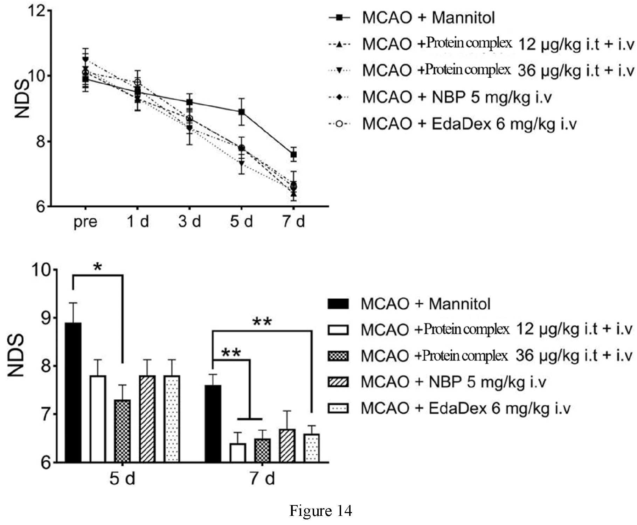

The neurological function of the model rats was evaluated by a blind method before and after the administration, and the results showed that:
after 5 to 7 days of administration, the neurological function of rats in the protein complex intrathecal combined intravenous administration group was superior to that of the control group, and there was no significant difference between the neurological function of either NBP administration group or EdaDex administration group and that of the control group (Figure 14).

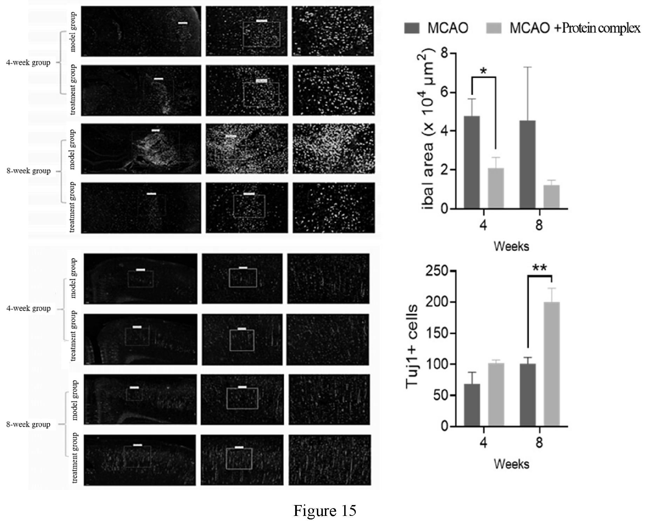

Neuroinflammation and neuronal markers were detected in the rat brain by immunofluorescence, respectively, and the results show that intrathecal combined intravenous administration of the protein complex suppressed the level of neuroinflammation and increased the number of neuron surviving (Figure 15).

The foregoing is a further detailed description of the present disclosure and is not to be regarded as a limitation of the particular embodiment of the disclosure. For a person of ordinary skill in the art to which the present disclosure belongs, a simple deduction or substitution without departing from the idea of the present disclosure is within the scope of protection of the present disclosure.

## Claims

1. A protein complex, **characterized in that** its production process comprises:
S1) culturing mesenchymal stem cells and creating a stressful environment using UV irradiation; and
S2) lysing the mesenchymal stem cells, followed by isolation and purification to obtain the protein complex.

2. The protein complex according to claim 1, **characterized in that** the protein complex comprises at least the following proteins:
sp|P02768|ALBU_HUMANSerum albumin OS=Homo sapiens;
sp|P02787|TRFE_HUMANSerotransferrin OS=Homo sapiens;
preferably, the mass of the above two proteins accounts for over 40% of the total protein complex mass;
preferably, the protein complex further comprises at least one of the following proteins:
sp|P51884|LUM_HUMAN Lumican OS=Homo sapiens;
sp|P62736|ACTA_HUMAN Actin, aortic smooth muscle OS=Homo sapiens;
sp|P01009|A1AT_HUMAN Alpha-1-antitrypsin OS=Homo sapiens;
sp|P07951|TPM2_HUMAN Tropomyosin beta chain OS=Homo sapiens;
sp|P08670|VIME_HUMAN Vimentin OS=Homo sapiens;
sp|P02751|FINC_HUMAN Fibronectin OS=Homo sapiens;
sp|P09493|TPM1 _HUMAN Tropomyosin alpha-1 chain OS=Homo sapiens;
sp|P21333|FLNA_HUMAN Filamin-A OS=Homo sapiens;
sp|P0DOX5|IGG1_HUMAN Immunoglobulin gamma-1 heavy chain OS=Homo sapiens;
sp|P24821|TENA_HUMAN Tenascin OS=Homo sapiens;
sp|P01023|A2MG_HUMAN Alpha-2-macroglobulin OS=Homo sapiens;
sp|P60709|ACTB_HUMAN Actin, cytoplasmic 1 OS=Homo sapiens;
sp|P69891|HBG1_HUMAN Hemoglobin subunit gamma-1 OS=Homo sapiens; and
sp|P01024|C3 HUMAN Complement C3 OS=Homo sapiens.

3. The protein complex according to claim 1, **characterized in that** the UV irradiation is conducted to stimulate the mesenchymal stem cells for 1 h to 30 h, preferably, for 10 h to 30 h, and more preferably for 6 h to 18 h;
preferably, the UV irradiation stimulation is conducted at an intensity of 10µW/cm² to 100µW/cm², and with a UV wavelength of preferably 290 nm to 340 nm;
preferably, a medium used during UV irradiation stimulation is serum-free MSCs medium.

4. The protein complex according to any one of claims 1 to 3, **characterized in that** the mesenchymal stem cells are selected from the group consisting of umbilical cord-derived human mesenchymal stem cells, bone marrow-derived mesenchymal stem cells, and human placental-derived mesenchymal stem cells.

5. A production process of a protein complex, comprising expanding MSCs, subjecting MSCs to a stress treatment through UV irradiation in culture, collecting the stress-treated MSCs for lysis treatment, and isolating and purifying proteins to obtain the protein complex.

6. The production process according to claim 5, **characterized in that** the UV irradiation is conducted to stimulate the mesenchymal stem cells for 1 h to 30 h, preferably, for 10 h to 30 h, and more preferably for 6 h to 18 h;
preferably, the UV irradiation stimulation is conducted at an intensity of 10µW/cm² to 100µW/cm², and with a UV wavelength of preferably 290 nm to 340 nm.

7. The production process according to claim 5 or 6, **characterized in that** a medium used during UV irradiation stimulation is serum-free MSCs medium.

8. Use of the protein complex according to any one of claims 1 to 4, wherein the use comprises use in the preparation of a medicament for the treatment of stroke.
